# EUROPEAN PATENT SPECIFICATION

(11) **EP 4 374 766 B1**
(45) Date of publication and mention of the grant of the patent: **02.07.2025**
(21) Application number: 22208874.2
(22) Date of filing: 22.11.2022
(51) Int. Cl.: A61B 1/00, A61B 1/005

(54) **ENDOSCOPE WITH CONTROL WHEEL AND VISUALIZATION SYSTEM**
ENDOSKOP MIT STEUERRAD UND VISUALISIERUNGSSYSTEM
ENDOSCOPE AVEC ROUE DE COMMANDE ET SYSTÈME DE VISUALISATION

(43) Date of publication of application: 29.05.2024
(73) Proprietor: Ambu A/S, 2750 Ballerup (DK)
(72) Inventor: GRAW, Felix Andreas, 86152 Augsburg (DE); EGGERS, Carolin, 21423 Drage (DE)
(74) Representative: AWA Denmark A/S

(56) References cited:
- JP-A- 2005 198 950
- JP-A- 2009 045 084
- JP-A- 2016 067 620
- KR-A- 20070 023 738
- US-A1- 2022 079 417

## Description

### TECHNICAL FIELD

The present disclosure relates to endoscopes, and in particular flexible endoscopes with steering control wheels.

### BACKGROUND

Endoscopes are known and used for visual navigation into, and examination and diagnosis of, hollow organs and body cavities, as well as, optionally, to assist in surgery, e.g. for a targeted tissue sampling. Endoscopes include procedure-specialized endoscopes, such as bronchoscopes, gastroscopes, colonoscopes and duodenoscopes. An endoscope provided with a flexible insertion cord generally includes an insertion tube, a bending section at a distal end of the insertion tube, and an observation optical system extending distally from the bending section. A control wheel is connected to the bending section by a steering wire. A user rotates the control wheel to bend the bending section. The ability to control direction of the distal end of the insertion cord facilitates navigation of the insertion cord of the endoscope through tortuous hollow organs and body cavities. Further, the user can easily observe an area of interest by directing an observation optical system arranged at a distal end portion of the insertion cord towards a target direction. An example of such endoscopes is described in e.g. WO21219832, which relates to a duodenoscope.

Procedures using endoscopes can be relatively time consuming, such as one hour or even longer, and may require some force from the user. For example, in case of a colonoscopy the user inserts the insertion cord of a colonoscope through the rectum and colon of a patient. Peristaltic movements of the colon may, however, work against forwarding the colonoscope, so the user must hold and push the insertion cord forward with one hand (typically the right hand) while holding the handle with the other hand (typically the left hand). Further, the user must at the same time operate the control wheel to direct the distal end of the insertion cord in a desired direction, which is typically done using fingers of the hand holding the handle (typically the left hand). Another example is insertion of a duodenoscope, e.g. for performing an Endoscopic Retrograde Cholangiopancreatography (ERCP), which is a very specialized and sensitive endoscopic procedure to diagnose and treat issues with the pancreatic duct, bile duct, and pancreas, where it is necessary to hold the duodenoscope in a specific position for an extended period at a treatment site, while potentially adjusting position of the distal end portion of the insertion cord by operating the control wheel, which again typically is done with fingers of the hand holding the handle (typically the left hand).

The user must be highly skilled and experienced, and as such is in high demand. The type of work is straining, intensive and repeated, which may give rise to repetitive strain injury. Further the ergonomics of the controls of known endoscopes are not ideal for all users. Users with relatively small hands may have difficulties in holding the endoscope handle and operating the endoscope controls using the same hand. This means that some users may have difficulties in performing their job or may face attrition over time.

EP1757217A1 suggests to provide a control wheel with larger radius, e.g. as an add-on part, and similarly US 2019/0320881 suggests an add-on wheel to increase the radius of a control wheel. Those suggestions may alleviate the above mentioned problems. However, the addition of components increases complexity and cost, which is undesirable, particularly in single-use endoscopes. JP2016067620A discloses a control wheel according to the preamble of independent claim 1 comprising protrusions having a base part and an extensions part wherein the extension part is extendable from a retracted position to an extended position so as to be adaptable to different hand sizes of the operator.

For single-use endoscopes, it is important that the entire device is manufactured in a cost-efficient way. For this reason, endoscopes are mainly made of polymeric materials to enhance disposability and reduce costs.

It is desirable to improve ergonomics of endoscopes, particularly in single-use endoscopes, to improve their value.

### BRIEF DESCRIPTION OF THE DISCLOSURE

The objective of the present disclosure is to provide an endoscope with features that eliminate or at least reduce the disadvantages of prior art endoscopes and suitably deal with the problems described above. In particular, it is an object of the present disclosure to present an endoscope, in particular a four-way endoscope, that exhibits improved ergonomics for persons with relatively small hands.

The objectives of the present disclosure are satisfied by an endoscope in accordance with claim 1 and by a visualization system including the endoscope and a monitor. Advantageous embodiments are claimed in the dependent claims and/or are explained herein below.

A first aspect of this disclosure relates to an endoscope comprising a proximal handle, and an insertion cord extending distally from the handle or interface, the insertion cord having distal bending section, a control wheel arranged at the handle, the control wheel having a wheel rotation axis and a perimeter comprising protrusions extending away from the wheel rotation axis, the control wheel controlling bending of the bending section by rotation of the control wheel around the wheel rotation axis, wherein at least one protrusion comprises a base part and an extension part which is extendable from a retracted position to an extended position.

By providing an endoscope as described above is achieved that the size of the control wheel may be adjusted, without the need for further parts. This ability to adjust may improve ergonomics for users and may further facilitate manipulation of the endoscope control wheel in that the force needed for rotating the control wheel may be smaller in an extended position of the extension part.

The extension part may be connected to the base part in any suitable way, for example detachably connected, such as configured to be moved from a socket corresponding to retracted position to a socket corresponding to an extended position. According to an embodiment, however, the extension part is connected to the base part via a joint. By joint is meant any connection or link allowing a turning or swinging motion between the extension part and the base part. An advantage is that the extension part is always connected to the base part, so that the extension part will not be lost, and operation is fool proof.

The joint may be any type of connection or link holding the parts together but allowing repositioning. However, according to the invention the joint is a turning joint. By turning joint is meant a connection or link allowing the extension part to be twisted from extending in a first direction to extending in a second direction, such as a joint connecting the base part to the extension part by a central tap or pin. A joint surface of the base part may be arranged at for example 45 degrees to a plane of the base part, and a corresponding joint surface of the extension part may be arranged at for example 45 degrees. An advantage of a turning joint is that further features and functions, e.g. locks or movement restrictions, may be built into the joint. As an example the joint surface of one part may be wavy, or have other surface features, and cooperating with the corresponding features of the other joint surface. Further the joint may be built-in and robust, as the joint can be formed without any exposed features.

In an alternative embodiment the joint is a hinge joint. The hinge joint can be a pin joint, where the extension part is connected to the base part at by a pin or tap to enable a rotational movement in a plane including the wheel rotation axis. This is a very intuitive solution for users.

An alternative embodiment uses a linear joint, wherein the extension part is telescopically connected to the base part, such as the extension part being housed inside the base part and slidably connected thereto. This can provide a robust joint.

As an alternative linear joint the extension part may be a telescopic spindle. Thus, the base part and extension part may be a screw pair, e.g. in that the base part comprises an internal thread and the extension part comprises an external thread, or vice versa. Such a joint will by nature have some protection against unintentional shift between retracted and extended position.

In some cases, it may be advantageous that the extension part is an L-shaped extension part configured for providing a projection protruding towards the handle, when the L-shaped extension part is in the extended position. This could further improve the reachability of the control wheel.

If considered relevant, the endoscope may further comprise an extension part lock releasably locking the extension part in the extended position. This may in some cases be advantageous to avoid accidental retraction of the extension part from the extended position during manipulation of the control wheel.

The endoscope may have any suitable number of protrusions, such as four or five, however according to an embodiment the control wheel comprises six protrusions, which is considered a favourable value to facilitate operation of the control wheel especially for users with relatively small hands.

The endoscope may have a simple solution, wherein only some of the protrusions, such as half of the protrusions comprise base part and extension part. According to an embodiment, however, all protrusions comprise base part and extension part, which from a user point of view is considered advantageous. The user then has the free choice whether one or the other or all protrusions should be extended.

According to an embodiment the retracted position corresponds to a radius rₘᵢₙ, and the extended position corresponds to a radius rₘₐₓ and satisfying a relationship rₘₐₓ = (rₘᵢₙ + Y mm), wherein Y denotes radius increase and advantageously having a value in the interval 6-12 mm, such as approximately 8 mm, which is found to provide improved ergonomics for a range of users. As an example rₘᵢₙ can be in the range of 50-60 mm, such as 54 mm, and rₘₐₓ can be in the range of 65-75 mm, such as 70 mm.

An aspect of the invention relates to a visualization system comprising an endoscope according to any one of the claims above and a monitor connectable to the endoscope.

A person skilled in the art will appreciate that any one or more of the above aspects of this disclosure and embodiments thereof may be combined with any one or more of the other aspects of this disclosure and embodiments thereof.

### BRIEF DESCRIPTION OF THE DRAWINGS

Embodiments of this disclosure will be described in more detail in the following with regard to the accompanying figures. The figures are not to be construed as limiting other possible embodiments falling within the scope of the attached claim set.
Fig. 1 is an illustration of a prior art endoscope,
Fig. 2 is an illustration of an endoscope with a control wheel according to a first embodiment,
Fig. 3a is a perspective view of a control wheel according to the invention in a first position,
Fig. 3b is a perspective view of the control wheel of Fig. 3a in a second position,
Fig. 3c is a plan view corresponding to Fig. 3a,
Fig. 3d is a plan view corresponding to Fig. 3b,
Fig. 3e is a perspective view of a part of the control wheel in Fig. 3a-3d,
Fig. 3f is a perspective view of another part of the control wheel in Fig. 3a-3d,
Fig. 4a is a perspective view of a control wheel according to a second embodiment in a first position,
Fig. 4b is a perspective view of the control wheel of Fig. 4a in a second position,
Fig. 4c is a plan view corresponding to Fig. 4a,
Fig. 4d is a plan view corresponding to Fig. 4b,
Fig. 5 is an illustration of an endoscope handle fitted with a control wheel according to the second embodiment
Fig. 6a is a schematic illustration of an alternative extension part according to an embodiment in a first position,
Fig. 6b is a schematic illustration of the extension part of Fig. 6a in a second position,
Fig. 7a-d are schematic illustrations of control wheels according to further embodiments,
Fig. 8a is a schematic illustration of an embodiment of a rotational extension part,
Fig. 8b is a schematic illustration of the embodiment of Fig. 8a in locked position,
Fig. 9a is a schematic illustration of an embodiment with another locking mechanism in a first position,
Fig. 9b is a schematic illustration of the embodiment of Fig. 9a in a second position, and
Fig. 10 is a schematic illustration of another locking mechanism.

### DETAILED DESCRIPTION

In the following the term "distal" refers to a position farthest away from the user, whereas the term "proximal" refers to the end closest to the user when using the endoscope in a patient.

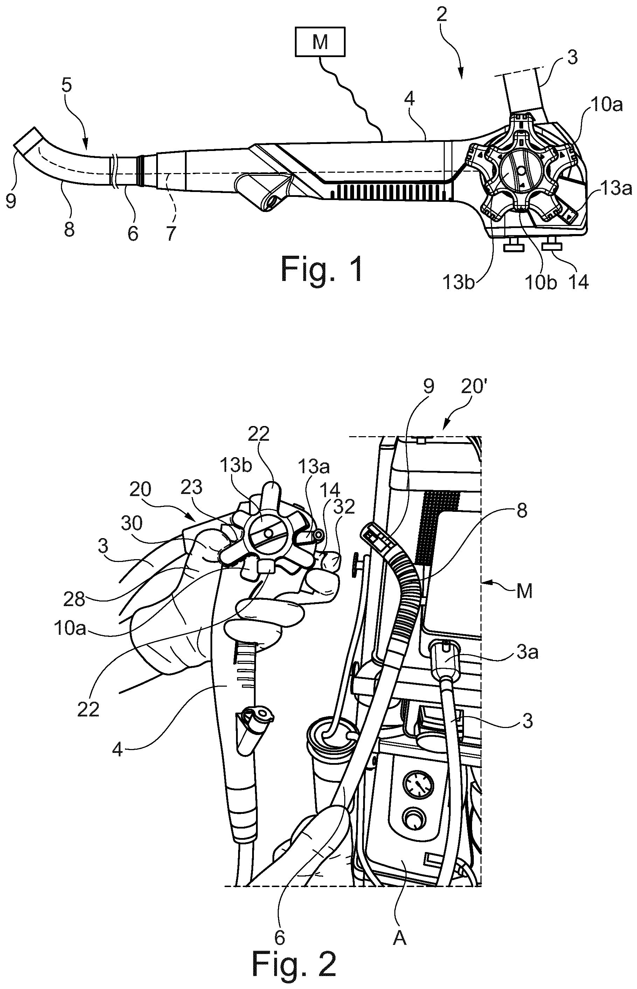

A prior art endoscope 2 is shown in Fig. 1. The endoscope 2 comprises an umbilical cord 3 connecting the endoscope 2 to further equipment, such as a vacuum source, an irrigation source, an electrical power source and a monitor M. The endoscope 2 comprises a handle 4 and an insertion cord 5 extending from the distal end of the handle 4. The insertion cord 5 comprises an insertion tube 6, a bending section 8, and a distal tip 9. The endoscope 2 is a 4-way endoscope, meaning that the bending section 8 may be controlled by rotating two control wheels 10a, 10b in different directions to move the distal tip 9 up/down and left/right. The control wheels 10a, 10b have a common rotation axis and are arranged on top of each other on the handle 4. The control wheels 10a, 10b are connected via steering wires 7 to the bending section 8. Bending the distal tip facilitate advancement of the insertion cord inside a patient. Rotation of the up/down control wheel 10a may be locked by up/down control wheel lock 13a, whereas rotation of the left/right control wheel 10b may be locked by the left/right control wheel lock 13b. The handle 4 also comprises a suction valve 14 as well as other valves and buttons.

Referring now to Fig. 2, an embodiment of a novel endoscope according to the disclosure, denoted by numeral 20, is presented. Unless otherwise noted, components having the same structure and/or function are denoted with the same numerals. Accordingly, the endoscope 20 comprises an umbilical cord 3 having a connector 3a connecting the endoscope to further equipment, such as the monitor M and potential auxiliary equipment A (such as irrigation water pump and vacuum source). The endoscope 20 and the monitor M may be referred to as a visualization system 20'. The endoscope 20 comprises the handle 4, the insertion cord 5, the control wheel 10a, and a control wheel 22 having a perimeter 23 and configured to bend the bending section 8 in the way that the control wheel 10b does.

The perimeter 23 comprises protrusions and valleys. By perimeter 23 is meant the outer contour of the control wheel 22 in plan view. The control wheel 10a is arranged closest to the handle, whereas the control wheel 22 is arranged outside the control wheel 10a. The control wheel 22 may bend the bending section left/right and the control wheel 10a may control the bending section up/down.

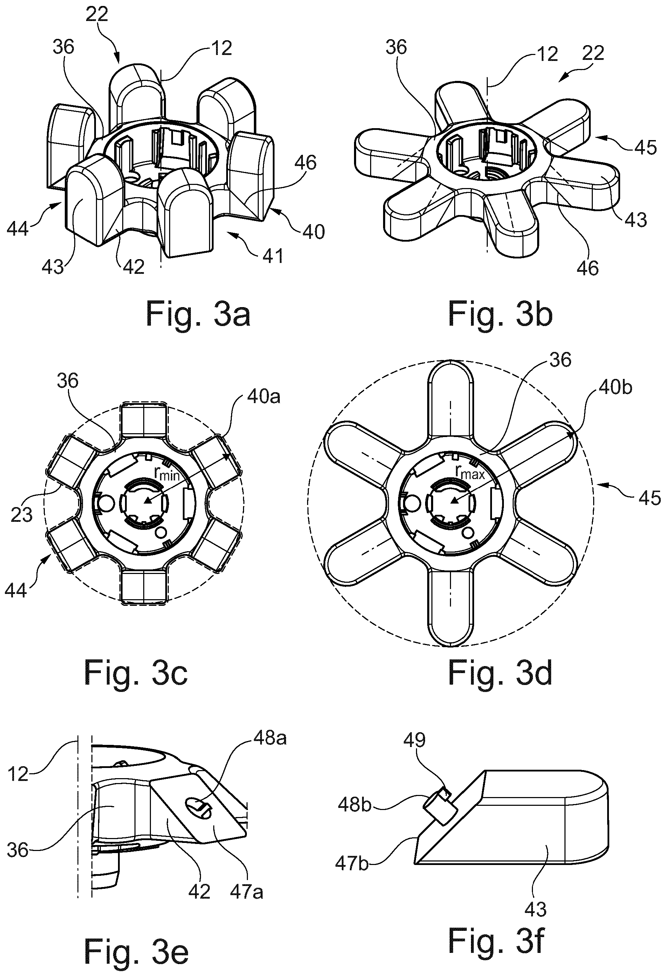

Fig. 2 shows a typical use situation of the endoscope 20, where the handle 4 is gripped by a left hand 28 of a user. A thumb 30 of the hand is positioned on the left/right control wheel 22, whereas an index finger 32 is positioned on a valve 14 of the endoscope to control e.g. suction of the endoscope. The hand illustrated in Fig. 2 is relatively large and has no difficulty in controlling the valve 14 and the left/right control wheel 22 at the same time. However, a user having a smaller hand may experience difficulties in reaching the left/right control wheel 22, which is positioned farthest away from the handle and further is smaller than the up/down control wheel 10a. The control wheel 22 is configured to overcome this problem. A first embodiment of the control wheel 22 is described in detail with reference to Figs. 3a-3f.

A control wheel 22 according to the invention is shown in Figs. 3a, 3b, 3c, 3d, 3e, and 3f. The control wheel 22 comprises protrusions 40 separated by valleys 41, and a hub 36. The protrusions 40 comprise a base part 42 and an extension part 43 connected at a turning joint 46. Fig. 3a is a perspective view illustrating a situation where all extension parts 43 are in a retracted position 44. In the retracted position 44 the extension parts 43 are arranged approximately parallel to the wheel rotation axis 12 providing a minimum extension of the protrusion 40 from the wheel rotation axis 12, so the extension parts 43 are inside a circle having a radius rₘᵢₙ, as also illustrated in the plan view of Fig. 3c, which also schematically illustrates the perimeter 23 in dashed line. The point of protrusion 40 farthest away from the wheel rotation axis 12 is in this case at a radius rₘᵢₙ. Depending on the shape of the base part 42 and the extension part 43, the point farthest away from the rotation axis 12 may be at the middle of the protrusion 40 or at one or both sides thereof. In the illustrated embodiment the point of the protrusion 40 farthest away from the rotation axis in Fig. 3c is at point 40a. A situation where all extension parts 43 are in an extended position 45 is illustrated in Fig. 3b. Here all extension parts 43 are twisted to extend approximately in radial direction from the wheel rotation axis 12 providing a maximum extension of the protrusion 40 from the wheel rotation axis 12. Fig. 3d is a plan view corresponding to the perspective view of Fig. 3b, and the point of protrusion 40 farthest away from the wheel rotation axis 12 is at a radius rₘₐₓ, so the perimeter 23 is changed compared to Fig. 3c. In the illustrated embodiment the point of the protrusion 40 farthest away from the rotation axis in Fig. 3c is at point 40a. Fig. 3e and 3f illustrate an exemplary embodiment of the turning joint 46. The turning joint comprises a base part 42 with a joint surface 47a arranged at an angle to the rotation axis 12. The angle may be e.g. 45 degrees. The base part 42 further comprises a joint keyhole 48a. The extension part 43 comprises a corresponding angled joint surface 47b configured to rotate on the angled joint surface 47a of the base part 42. The extension part 43 further comprises a joint key 48b with a key projection 49 adapted for engaging the keyhole 48a of the base part 42. Hence the base part 42 and the extension part 43 may be connected in a turning joint 46 with one degree of freedom by inserting the joint key 48b of the extension part 43 into the keyhole 48a. The key projection 49 can ensure that the extension part 43 of the turning hinge 46 can turn relatively to the base part 42, but not disconnect (except in a special position, where the key projection 49 register with a corresponding opening of the keyhole 48a).

The hub 36 and base part 42 may be of unitary construction, such as made of plastic and moulded in one piece. The extension part 43 including the key projection 49 may also be moulded in one piece in a plastic material. The plastic material may be for example ABS or PP. A bioplastic material may also be used.

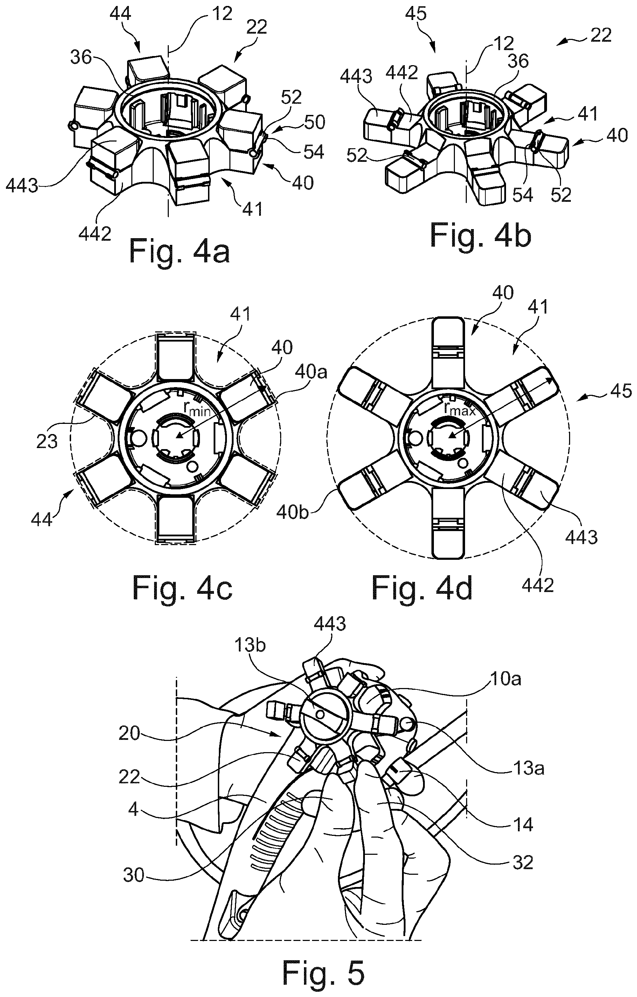

A second embodiment of a control wheel 22 according to the present disclosure is shown in Figs. 4a-4d. The control wheel comprises protrusions 40, valleys 41, and a hub 36. The protrusions 40 comprise a base part 442 and an extension part 443, which are interconnected by a hinge joint 50. The hinge joint 50 comprise a hinge tap 52 and hinge grippers 54. Fig. 4a illustrate a situation where all extension parts 443 are in a retracted position 44 in that extension parts 443 are folded back on top of the base parts 442. The extension parts 443 are inside a circle having a radius rₘᵢₙ and length of the protrusion 40 in radial direction from the wheel rotation axis 12 is minimum, as also illustrated in the plan view of Fig. 4c. The point of the perimeter 23 farthest away from the wheel rotation axis 12 in the illustrated embodiment of Fig. 4c is at point 40a.

A situation where all extension parts 443 are in an extended position 45 is illustrated in Fig. 4b. Here all extension parts 443 are folded out about the hinge joint 50 to extend approximately in radial direction from the wheel rotation axis 12 providing a maximum extension of the protrusion 14 from the wheel rotation axis 12, as also seen in the plan view of Fig. 4d. The extension parts 443 are inside a circle having a radius rₘₐₓ and length of the protrusion 40 in radial direction from the wheel rotation axis 12 is maximum, as illustrated in the plan view of Fig. 4d. The perimeter 23 of the control wheel has changed when comparing Fig. 4c to 4d, and the point of the perimeter 23 farthest away from the wheel rotation axis 12 in the extended position 45 in the illustrated embodiment of Fig. 4d is at point 40b.

The hinge may have a separate key, tap or shaft to turn around, which will facilitate production, as the parts can be simple. For single use it is however preferred that the endoscope is made of few parts, which facilitates assembly. Further it is advantageous that the parts are made of e.g. plastic, to facilitate mass production e.g. by moulding. By moulding it is possible to provide parts with integrated hinge elements.

An endoscope 20 fitted with a control wheel 22 according to the second embodiment illustrated in Figs. 4a-4d is shown in Fig. 5. The endoscope handle 4 is held by a user in the left hand, whereas index finger 30 and thumb 32 of right hand is used to extend one of the extension parts 443. Some extension parts 443 are in the retracted position, whereas others are in the extended position. Left hand can operate the suction valve 14 and normally also the up/down control wheel rotation lock 13a, whereas the left/right control wheel rotation lock 13b generally needs to be operated by the right hand of the user.

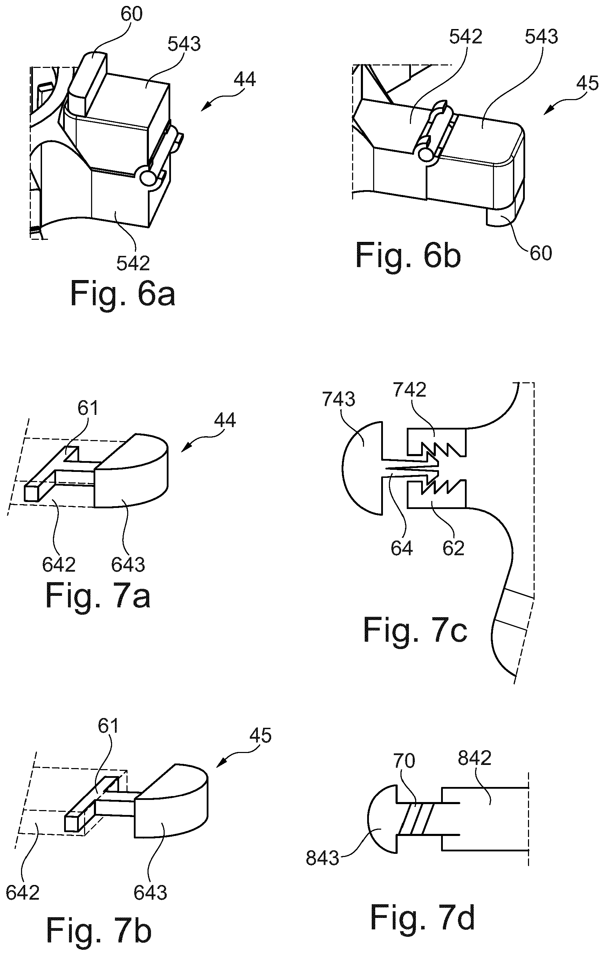

An L-shaped extension part 543 according to an embodiment is shown in Fig. 6a and 6b, which are close-ups of protrusions similar to the protrusions 40 shown in Fig. 4a and 4b. The L-shaped extension part 543 is similar to the extension part 443 of Fig 4 but comprises a projection 60. Fig. 6a illustrate a retracted position 44 of the extension part 543, which is folded on top of the base part 542, and the projection 60 extends upwards in the figure. Fig. 6b illustrates the extended position 45 of the L-shaped extension part 543 in which the extension part 543 is folded out, and the projection 60 protrudes in axial direction downwards towards the handle (not shown). The extension part 543 is connected to a base part 542 by a hinge.

Alternative embodiments using a telescopic connection of the extension part to the base are shown in Figs. 7a to 7d. Of these Figs. 7a and 7b are partly x-ray illustrations schematically illustrating an embodiment, where the extension part 643 is slidably connected to the base part 642 via a slider 61 of the extension part 643 engaging a groove (not shown) in the interior of the base part 642. The retracted position 44 is shown in Fig. 7a, whereas the extended position 45 is shown in Fig. 7b. The schematic sectional view of Fig. 7c illustrates an alternative telescopic connection according to another embodiment. Here the extension part 743 comprises a resilient leg 64 engaging a ratchet 62 inside the base part 742. A further alternative telescopic connection is shown in the schematic sectional view of Fig. 7d. The extension part 843 is a telescopic spindle 70 having threads engaging corresponding threads of the base part 842. The user may extend the extension part 843 by turning the extension part relatively to the base part 842. Suitably the telescopic connection of the extension part is provided with an end stop to avoid disengagement of the extension part from the base part.

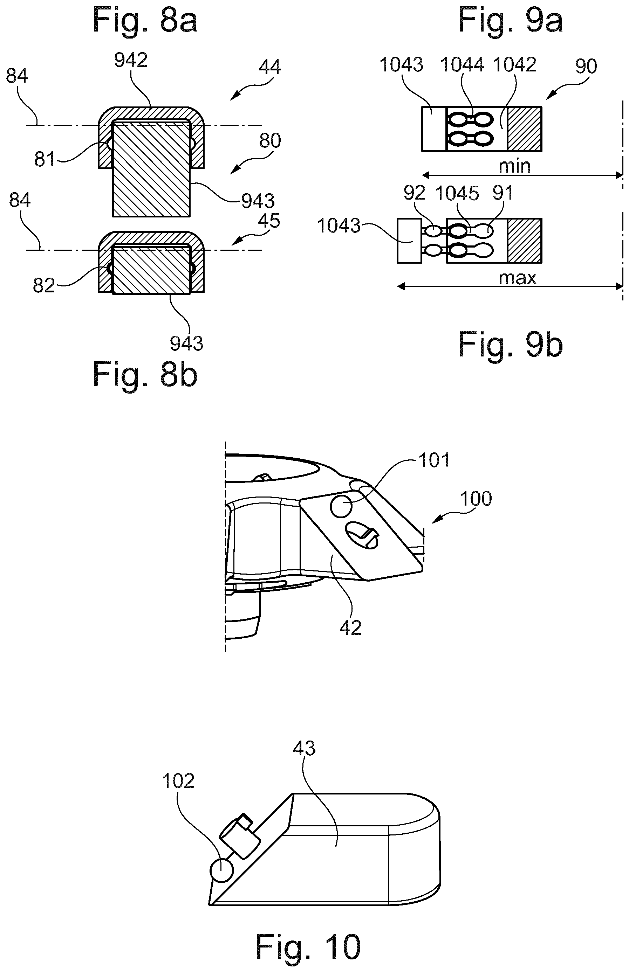

The endoscope may further comprise a releasable extension part lock to prevent or minimize the risk of unintentional shift between the extended position 45 and the retracted position 44. Suggested extension part locks are schematically illustrated in Figs. 8-10. In the example illustrated in Fig. 8a and 8b an extension part 943 is rotatably connected to base part 942 with a rotation axis 84 with the extension part 943 arranged inside base part 942. Fig. 8a illustrates a retracted position 44 of the extension part 943, whereas Fig. 8b illustrates an extended position 45 of the extension part 943, which in this case is rotated to extend out of the plane of the paper. The extension part 943 comprises bosses 82, which in the extended position 45 engage corresponding recesses 81 formed in the base part 942 to form an extension part lock 80. An alternative extension part lock 90 is illustrated in Fig. 9a and 9b. An extension part 1043 comprises legs 1044 engaging corresponding openings 1045 of a base part 1043. The legs 1044 of the extension part 1043 comprise bulbs 92 engaging recesses 91 of opening 1045, thereby restricting movement of the extension part 1043 relatively to the base part 1042. The schematically illustrated lock 90 limits the risk of unintentional movement of the extension part 1043 from the retracted position at rₘᵢₙ to the extended position at rₘₐₓ and vice versa, as the bulbs 92 rest in the recesses 91. Fig. 10 schematically illustrate an extension part lock 100 for a construction similar to Fig. 3. The extension part lock 100 comprises a recess 101 and a corresponding boss 102, which will register and lock when the extension part 43 is in the extended position.

The elements may be made in any suitable material. For single use focus is on mass production, and there is not a need for harsh cleaning and sterilization of the endoscope after use (as it not to be reused), meaning that the elements and parts of the endoscope can be made of a plastic material, such as ABS, PP or bioplastics, which may be moulded, extruded or 3-D printed to the desired shape in high numbers. The number of parts may be kept low, meaning that assembly is facilitated. The use of metal can be kept low, which enables a low carbon footprint of the endoscope.

### List of reference signs:

2 Endoscope
3 Umbilical cord
3a connector
4 handle
5 insertion cord
6 insertion tube
7 steering wires
8 bending section
9 distal tip
10 control wheel
10a up/down control wheel
10b left/right control wheel
12 wheel rotation axis
13a up/down control wheel rotation lock
13b left/right control wheel rotation lock
14 suction valve
20 endoscope
20' visualization system
22 control wheel
23 perimeter
28 hand
30 thumb
32 index finger
36 hub
40 protrusion
40a protrusion point
40b protrusion point
41 valley
42, 442, 542, 642, 742, 842, 942, 1042 base part
43, 443, 543, 643, 743, 843, 943, 1043 extension part
44 retracted position
45 extended position
46 turning joint
47a, 47b joint surface
48a joint keyhole
48b joint key
49 key projection
50 hinge joint
52 hinge tap
54 tap gripper
60 extension projection
61 slider
62 ratchet
64 resilient leg
70 telescopic spindle
80, 90, 100 extension part lock
81 recess
82 boss
91 recess
92 bulb
101 recess
102 boss
1044 leg
1045 opening
M monitor
A auxiliary equipment
rₘᵢₙ minimum radius
rₘₐₓ maximum radius

## Claims

1. Endoscope (20) comprising
a proximal handle (4) or interface, and
an insertion cord (5) extending distally from the handle or interface, the insertion cord having distal bending section (8),
a control wheel (22) arranged at the handle, the control wheel having a wheel rotation axis and a perimeter (23) comprising protrusions (40) extending away from the wheel rotation axis,
the control wheel controlling bending of the bending section by rotation of the control wheel around the wheel rotation axis,
wherein at least one protrusion comprises a base part (42) and an extension part (43) wherein the extension part is connected to the base part via a turning joint (46), wherein the extension part is extendable from a retracted position (44) to an extended position (45)
**characterized in that** in the retracted position the extension part is arranged approximately parallel to the wheel rotation axis and in the extended position the extension part is twisted to extend approximately in a radial direction from the wheel rotation axis.

2. Endoscope according to claim 1, further comprising an extension part lock (80, 90) releasably locking the extension part in the extended position.

3. Endoscope according to claim 1 or 2, wherein the control wheel comprises six protrusions.

4. Endoscope according to any one of the claims above, wherein all protrusions comprise base part and extension part.

5. Endoscope according to any one of the claims above, wherein the retracted position corresponds to a radius rₘᵢₙ, and the extended position corresponds to a radius rₘₐₓ and satisfying a relationship rₘₐₓ ≥ (rₘᵢₙ + Y mm), wherein Y denotes radius increase and advantageously having a value in the interval 6-12 mm, such as approximately 8 mm.

6. Visualization system comprising an endoscope according to any one of the claims above and a monitor (M) connectable to the endoscope.

## Patentansprüche

1. Endoskop (20), umfassend:
einen proximalen Griff (4) oder eine proximale Schnittstelle und
eine Einführschnur (5), die sich distal von dem Griff oder der Schnittstelle erstreckt, wobei die Einführschnur einen distalen Biegeabschnitt (8) aufweist,
ein Steuerungsrad (22), das an dem Griff angeordnet ist,
wobei das Steuerungsrad eine Raddrehachse und einen Umfang (23) aufweist, der Vorsprünge (40) umfasst, die sich von der Raddrehachse weg erstrecken,
wobei das Steuerungsrad das Biegen des Biegeabschnitts durch Drehen des Steuerungsrads um die Raddrehachse steuert,
wobei mindestens ein Vorsprung einen Basisteil (42) und einen Verlängerungsteil (43) umfasst, wobei der Verlängerungsteil über ein Drehgelenk (46) mit dem Basisteil verbunden ist, wobei der Verlängerungsteil aus einer zurückgezogenen Position (44) in eine ausgefahrene Position (45) ausfahrbar ist,
**dadurch gekennzeichnet, dass** der Verlängerungsteil in der zurückgezogenen Position ungefähr parallel zu der Raddrehachse angeordnet ist und der Verlängerungsteil in der ausgefahrenen Position verdreht ist, um sich ungefähr in einer radialen Richtung von der Raddrehachse zu erstrecken.

2. Endoskop nach Anspruch 1, ferner umfassend eine Verlängerungsteilverriegelung (80, 90), die den Verlängerungsteil in der ausgefahrenen Position lösbar verriegelt.

3. Endoskop nach Anspruch 1 oder 2, wobei das Steuerungsrad sechs Vorsprünge umfasst.

4. Endoskop nach einem der obigen Ansprüche, wobei alle Vorsprünge Basisteil und Verlängerungsteil umfassen.

5. Endoskop nach einem der obigen Ansprüche, wobei die zurückgezogene Position einem Radius rₘᵢₙ entspricht und die ausgefahrene Position einem Radius rₘₐₓ entspricht und eine Beziehung rₘₐₓ ≥ (rₘᵢₙ + Y mm) erfüllt, wobei Y eine Radiuszunahme bezeichnet und vorteilhafterweise einen Wert in dem Intervall von 6 - 12 mm, wie ungefähr 8 mm, aufweist.

6. Visualisierungssystem, umfassend ein Endoskop nach einem der obigen Ansprüche und einen mit dem Endoskop verbindbaren Monitor (M).

## Revendications

1. Endoscope (20) comprenant
une poignée proximale (4) ou une interface, et
un cordon d'insertion (5) s'étendant de manière distale à partir de la poignée ou de l'interface, le cordon d'insertion présentant une section de flexion distale (8),
une molette de commande (22) agencée au niveau de la poignée, la molette de commande présentant un axe de rotation et un périmètre (23) comprenant des protubérances (40) s'étendant à l'opposé de l'axe de rotation de la molette,
la molette de commande commandant la flexion de la section de flexion par rotation de la molette de commande autour de l'axe de rotation de la molette,
dans lequel au moins une protubérance comprend une partie de base (42) et une partie d'extension (43), dans lequel la partie d'extension est reliée à la partie de base par le biais d'un joint tournant (46), dans lequel la partie d'extension peut être étendue d'une position rétractée (44) à une position étendue (45)
**caractérisé en ce que** dans la position rétractée la partie d'extension est agencée approximativement parallèlement à l'axe de rotation de la molette et dans la position étendue la partie d'extension est tordue pour s'étendre approximativement dans une direction radiale à partir de l'axe de rotation de la molette.

2. Endoscope selon la revendication 1, comprenant en outre un verrou de partie d'extension (80, 90) verrouillant de manière libérable la partie d'extension dans la position étendue.

3. Endoscope selon la revendication 1 ou 2, dans lequel la molette de commande comprend six protubérances.

4. Endoscope selon l'une quelconque des revendications précédentes, dans lequel toutes les protubérances comprennent une partie de base et une partie d'extension.

5. Endoscope selon l'une quelconque des revendications précédentes, dans lequel la position rétractée correspond à un rayon rₘᵢₙ, et la position étendue correspond à un rayon rₘₐₓ et satisfait à une relation rₘₐₓ ≥ (rₘᵢₙ + Y mm), où Y désigne l'augmentation du rayon et a avantageusement une valeur dans l'intervalle de 6 à 12 mm, telle qu'environ 8 mm.

6. Système de visualisation comprenant un endoscope selon l'une quelconque des revendications précédentes et un moniteur (M) pouvant être connecté à l'endoscope.
